# EUROPEAN PATENT APPLICATION

(11) **EP 3 539 516 A1**
(43) Date of publication of application: **18.09.2019**
(21) Application number: 18382993.6
(22) Date of filing: 26.12.2018
(51) Int. Cl.: A61F 5/448

(54) **HOLDING DEVICE FOR AN OSTOMY PATCH**

(30) Priority: 03.01.2018 ES 201830004 U
(71) Applicant: Rivas Vicente, Carolina, Molina de Segura, Murcia (ES); Desiderio Gil, Jose Antonio, Molina de Segura, Murcia (ES)
(72) Inventor: Rivas Vicente, Carolina, Molina de Segura, Murcia (ES); Desiderio Gil, Jose Antonio, Molina de Segura, Murcia (ES)

(57) **Abstract**

Holding device (1) of a patch (2) for ostomies, that comprises a holding element (9) longitudinal, that presents a laminar flat shape, with a first and a second ending (10.1, 10.2) opposed; two arms (11) longitudinal with flat laminar shape, where both arms (11) are arranged symmetrically and are suitable to fit between the second side (3.2) of the patch (2) and the peripheral area (8) of the circular element (6), and; an union means of the first ending (10.1) of the holding device (9) with the first ending (12.1) of union of both arms (11), suitable to permit a first position of use in which the holding element (9) and both arms (11) are contained in a same plane and present a same symmetry axis, being the second ending (10.2) of the holding device (9) opposed to the second ending (12.2) of the arms (11).

## Description

### Scope of the invention

The present invention corresponds to the technical field of the care elements for the ostomies cares, made by a patch or adhesive element and a picking bag, where such patch is made by a flat body with a first and second opposed sides and a central orifice, where the first side presents an adhesive and it is apt to fix to the skin of the patient abdomen and the second side comprises an structure suitable to hold the picking bag.

### Background of the invention

An ostomy is a surgical bypass of a hollow viscera or a conduit, generally the windpipe, the intestine or the urinary tract, to make it appear in the surface of the skin. In the case of digestive and urologic ostomies, it is made an artificial communication of an organ with the abdominal wall by means of a surgical procedure, with the goal of substitute the natural ways of excretion.

When an ostomy is done to a patient, the internal conduit of the body becomes external, and the patient needs a special control and monitoring of the stoma, which is the opening made in the body joining a part of the body cavity with the outer. Such control and monitoring consists so much in the caring of such ending of the external conduit, as in the control of the picking of stool or urine where appropriate, which go out by such ending of the conduit, in the most discrete way as possible, so the patient can continue with a life closer to the normal life.

To the control of the going out to the outer of the body wastes it is used a bag for the picking up of the same. This bag presents an opening to empty it each time it is necessary, avoiding in this way a very frequent substitution of the bag, so such substitution is made in a pre-set time, but longer than what implies a change after each filling. The goal is trying to avoid injures in the skin and favours a lower dependence of the cures.

The picking bag needs some means that allow it fixing around the stoma of the patient. To this effect, it is used an adhesive element or patch placed with the face of the same which contains adhesive material on the abdomen of the user, around the stoma.

Similarly, the patch presents in the opposite side to the adhesive one, a holding element for the bag.

In this type of cares it is important, furthermore of the change of the bag before it looses or some leakage occurs, to look out for making, from time to time, the change of the patch that keep it fixed to the skin.

Such adhesive patch remains sealed to the skin during some time that depends on a lot of factors, as the suitable sizing, the climate, the skin condition, the scars, the change in the weight, the feeding, the activity and the shape of the body near the stoma. Thus, the sweating, the oily and moist skin, the changes of weight... reduce the number of days that the adhesive remains correctly stuck. The body heat, together with the room temperature, provokes the barriers for the skin get loose faster than usual.

Of course, another factor that influences and much in a higher effectivity time of the adhesive is making a correct stuck of the same, without making folds of the same on the skin.

At the present time, it is noticed an important inconvenient in this type of patches because when the patient takes the patch on his or her hand and takes out the protection paper of the adhesive, he or she much take the patch with the fingers of both hands so it can be extended and it is possible to place it correctly in the corresponding area. Hence, with this way of holding the patch, although trying to avoid it, it is really very complicated to place it without the fingers touching the adhesive of the same.

Considering that the patch presents the precise amount of adhesive that permits performing its function without affecting the skin of the patient, this simple gesture of contact of the fingers with the adhesive affects harmfully the capacity of adhesion of the same, because part of the same remains in the fingers of the patient. This entails that when the patients achieve fitting the patch, feels that this doesn't have the expected adherence. This provokes stress in the patient, who must repeat the process several times until a suitable fitting is achieved.

On the other side, a repetition of the placing process means a stuck and unstuck of the same, which reduces the adhesive properties on each attempt, so the patient finally throw out the patch and takes a new one.

Furthermore, although a correct hygiene is necessary at the moment of changing the patch, it is a reality that in many cases the patients have traces of grease in the fingers, general due to the own body grease. This is also an inconvenient, because when the fingers get into contact with the adhesive, part of the grease is stuck to the same, which reduces even more the adherent properties of the adhesive.

No element or device has been found in the estate of art to be intended to the fitting of patch for ostomies and allows the process of the patch adhesion in the abdomen of the patient, easily, avoiding the contact of the user fingers with the adhesive.

### Description of the invention

The holding device for an ostomy patch here described, refers to a device for holding such patch at the moment of placing it, being such patch made by a flat body with a first and second opposite sides and a first central hole. The fist side of this patch presents an adhesive and it is apt to fix it to the skin of the abdomen of the patient and the second side comprises a means suitable to the holding of the picking bag, fixed around the first hole and formed by a circular element with a second central hole concentric with the first hole and a peripheral area separated from the second side of the patch along all the outline of the circular element, which is defined between an outer diameter and an intermediate diameter of such circular element.

This holding device comprises a longitudinal holding element, which presents a flat laminar shape, with a first and second opposed endings.

Furthermore, it comprises both longitudinal arms with flat laminar shape, presenting a first ending for joining both arms and a second free ending, where both arms are disposed symmetrically and are suitable to fit themselves between the second side of the patch and the peripheral area of the circular element, being the separation area between such second free ending equal or higher than the intermediate diameter of the circular element and smaller that the outer diameter of the same.

It also presents an union means of the first ending of the holding device with the firs ending of union of both arms, suitable to permit a first position of use in which the holding element and both arms are contained in a same plane and present a same symmetry axis, being the second ending of the holding device opposed to the second ending of the arms.

According to a preferred embodiment, the union means are made by a groove placed in the first ending of the holding element, where such groove has a straight shape perpendicular to the symmetry axis, and a length equal to the width of the holding element so the holding device presents a second position in which the holding element is arranged with a turn of 180° regarding the first position and placed on the longitudinal arms, contained in the parallel plane to the same.

In another preferred embodiment, the union means are made by two longitudinal openings arranged in both arms respectively next to the first union ending of both and, tow tabs arranged in both laterals of the same ending of the holding element, apt to couple in one of the openings of the arms respectively.

According to a preferred embodiment, both arms have a straight parallel section each other, from a section next to the first ending until the second free ending.

In another preferred embodiment, both arms present a shape of circular arch.

With the holding device for an ostomy patch here presented it is obtained a significant improvement of the state of art.

This is because with this device it is possible to fit such patch by the patient, easily, without the patient touching with the fingers the area of the adhesive of the same.

It is a device very easy to use in patients of any age, and achieving the avoidance of the fingers contact, it is obtained a perfect adhesion of the patch.

Furthermore, it is achieved a correct hygiene of the placing process of the patch, because there is not direct contact of the patient with the same.

It results, in addition, a very practical device because it is possible to reduce the size it occupies in as much as the holding element can be separated from the rest of the body of the device or, can be folded over the same, reducing in this way the dimensions of the device. This favours the possibility of including a device inside each patch box, so the sale can be done together the same and due to its reduced costs, the patch will not suffer a price increase and all the patients can have access to a holding device.

All of this produces a very easy, practical and furthermore economic and very efficient device.

### Brief description of the drawings.

With the purpose of helping to a better understanding of the characteristics of the invention, according to an example of a preferred embodiment of practical realization, it is given, as integral part of such description, a series of drawings where, illustrative but not limitative it has been represented the following:
Figures 1.1 and 1.2.- Show the lower and upper views of the patch for ostomies
Figures 2.1, 2.2 and 2.3.- show plant, profile views and of a detail A respectively, of the holding device in a first position of use, for a first preferred embodiment of the invention.
Figure 3.- Shows a plant view of the holding device in a second position, for a second preferred embodiment of the invention.
Figure 4.- Shows a plant view of the holding device with the arms fit in the suitable means for holding the picking bag of the patch, for a first preferred embodiment of the invention.
Figure 5.- Shows a plant view of the holding device in a first position of use, for a second preferred embodiment of the invention.
Figure 6.- Shows a perspective view of the holding device with the holding element separated from the arms of the device, for a first preferred embodiment of the invention.

### Detailed description of a preferred embodiment of the invention

In the view of the drawings given, it can be observed as in a first preferred embodiment of the invention, the holding device (1) of a patch (2) for ostomies here proposed, is intended for the placing of such patch (2) comprised by a flat body with a first and second sides (3.1, 3.2) opposed from each other and a first central (4) hole.

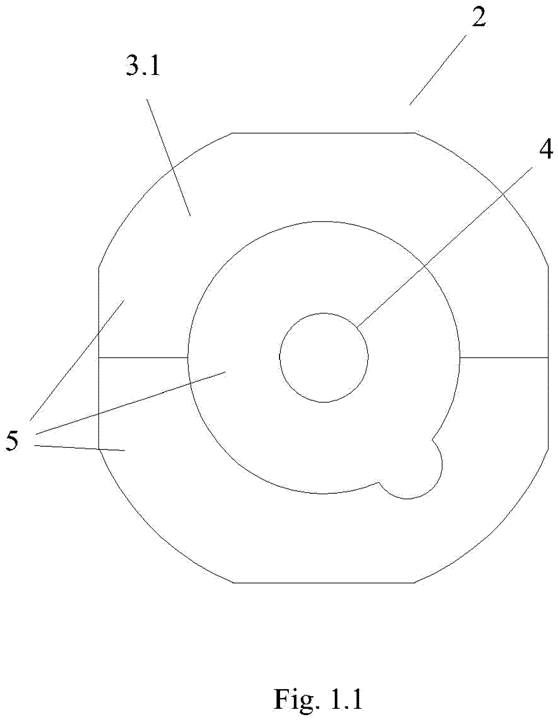

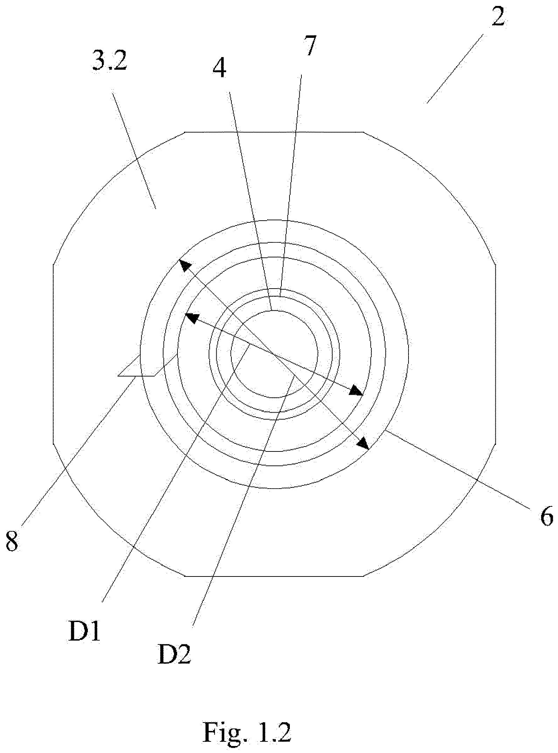

As it is showed in Figures 1.1 and 1.2, the first side (3.1) of this patch (2) presents an adhesive, with a protection lay (5) of the same, and it is suitable to get fixed to the skin of a patient abdomen while the second side (3.2) comprise suitable means to the holding of a picking bag (not represented in the Figures), fixed around the first hole (4) and formed by a circular element (6) with a second central (7) hole concentric with the first hole (4) and a peripheral area (8) separated from the second side (3.2) of the patch (2) along all the outline of the circular element (6), which is defined between an outer diameter (D2) and an intermediate diameter (D1) of such circular element (6).

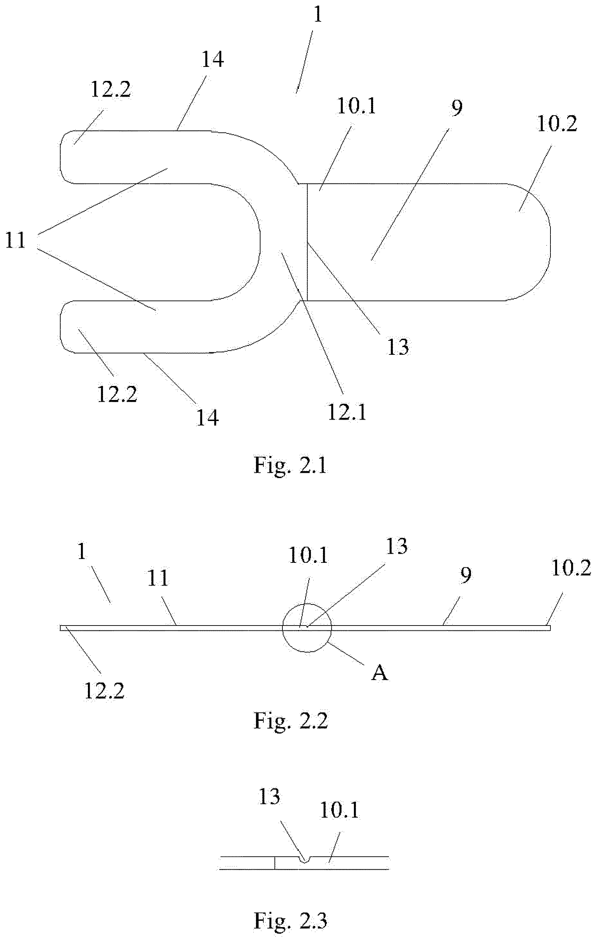

As can be observed in the Figures 2.1, 3 and 4, this holding device (1) comprise a holding element (9) longitudinal, which presents a flat laminar shape, with a first and a second ending (10.1, 10.2) opposed.

Also, the holding device (1) comprise two arms (11) longitudinal in flat laminar shape. In the Figures 2.1, 3 and 4 it is showed that both arms (11) present a first ending (12.1) of union of the same and a second ending (12.2) free and are arranged symmetrically.

It is meet the condition that the separation space between the second endings (12.2) free of each one of such arms (11) is bigger or equal than the intermediate diameter (D1) of the circular element (6) and smaller than the outer diameter (D2) of the same, so they are suitable to fit between the second side (3.2) of the patch (2) and the peripheral area (8) of the circular element (6).

The holding device (1) presents furthermore a union means of the first ending (10.1) of the holding element (9) with the first ending (12.1) of union of both arms (11). Such union means are suitable to allow a first position of use of the device, in which the holding element (9) and both arms (11) are contained in a same plane and present a same symmetry axis, being the second end (10.2) of the holding element opposed to the second ending (12.2) of the arms (11).

In this first preferred embodiment of the invention, these union means are made by a groove (13) placed in the first ending (10.1) of the holding element (9).

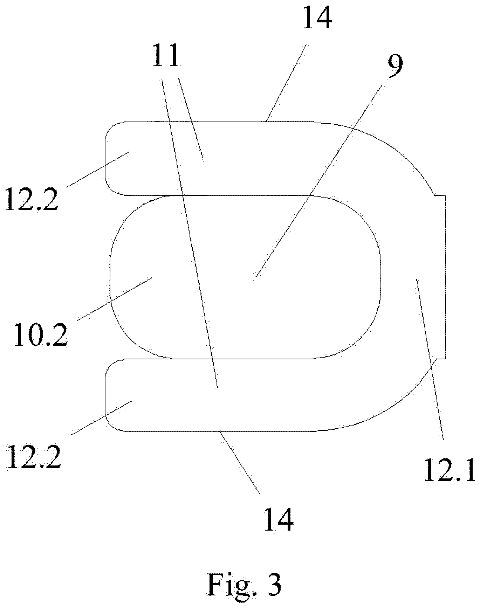

This groove (13) as showed in the Figure 2.1, has a straight shape perpendicular to the symmetry axis and a length equal to the width of the holding element (9). This groove (13) furthermore, as can be observed in the Figures 2.2 and 2.3 has a determined depth so it allows the turn of the holding element (9) on the arms (11), so the holding device (1) adopts a second position in which such holding element (9) is arranged with a turn of 180° with respect to the first position and it is arranged on the arms (11) longitudinal, containing in a second parallel plane to the same, such as it is showed in the Figure 3.

Furthermore, in this first preferred embodiment, as it can be observed in the Figures 2.1, 3 and 4, the arms (11) of the device present a straight section (14) parallel between them, from a first section next to the first ending (12.1) until the second free ending (12.2).

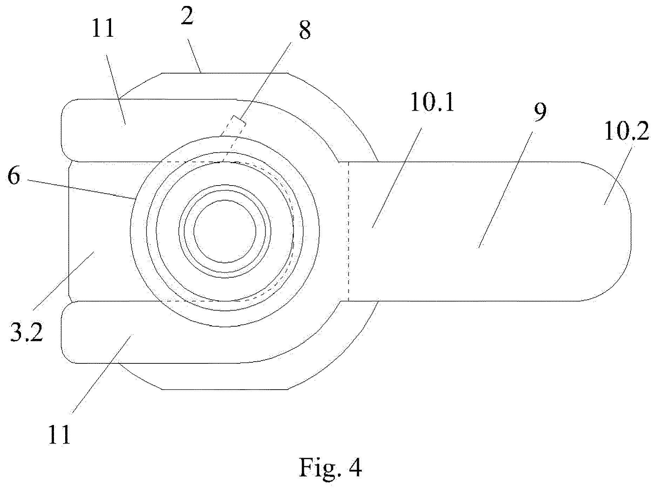

Therefore, as it is showed in the Figure 4, when the patient is ready to fit a patch (2) on the stoma, both arms (11) of the device get fit between the second side (3.2) of the patch (2) and the peripheral area (8) of the circular element (6) that conforms the means suitable to hold a picking bag. On this way, the patient can handle the patch (2) using the device, taking it by the holding element (9) of the same, without having contact with the adhesive of the first side (3.1) of the patch (2).

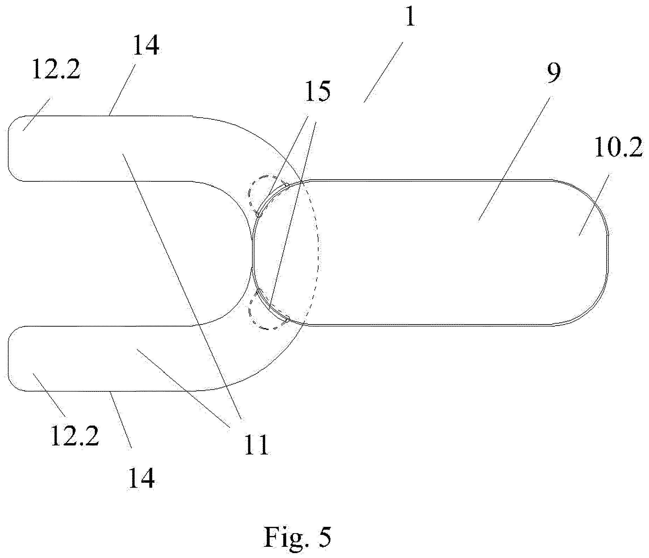

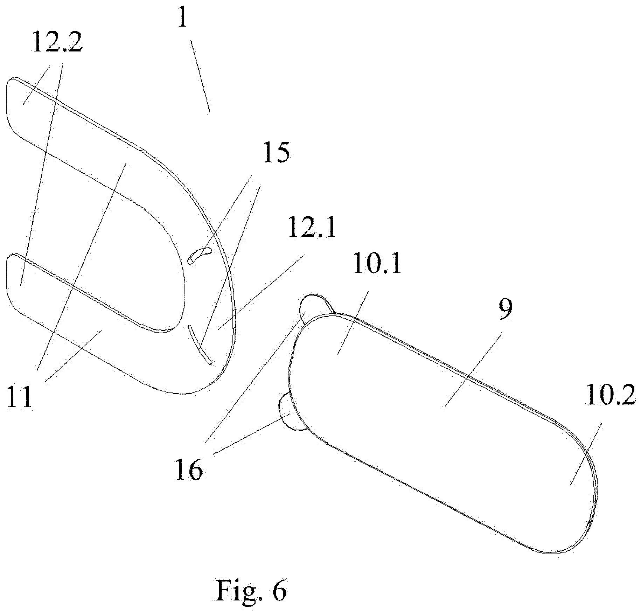

In the present memory a second preferred embodiment of the invention is presented, in which, differently to the first proposed embodiment, and such as it is showed in the Figures 5 and 6, the union means between the first ending (10.1) of the holding element (9) and the first ending (12.1) of union of both arms (11) of the device are made by two openings (15) longitudinal, arranged in both arms (11) respectively next to the first ending (12.1) of union of both and, two tabs (16) arranged in both laterals of the first ending (10.1) of the holding element (9) apt to couple on echa one of the openings (15) longitudinal of the arms (11) respectively.

Such union means of this second preferred embodiment are equally apt to permit a first position of use of the device, in which the holding element (9) and both arms (11) are contained in a same plane and present a same symmetry axis, being the second ending (10.2) of the holding element (9) opposed to the second ending (12.2) of the arms (11). So, this first use position is showed in Figure 5 and in that it can be observed that both tabs (16) of the first ending (10.1) of the holding element (9) are coupled in the groove (15) corresponding of one of the arms (11).

These union means of this second embodiment permit equally that when the device is not in the first position of use, that is, the tabs (16) are not coupled in the corresponding longitudinal openings (15), the holding element (9) is separated from the arms (11) of the device.

Therefore, in any of the proposed embodiments it is possible to adopt a first position of use and furthermore, when the device is not in such position, they also permit a reduction of the space they occupy, so that it is possible it storage in a reduced space, as it can be seen in the package of the own patches.

The embodiment described are only examples of the present invention, so, the details, terms and specific sentences used in the present memory cannot be considered as limitative, but they must be understood only as a base for the claims and as a representative base that supplies a comprehensive description as the enough information to the expert in the field to apply the present invention.

With the holding device of a patch for ostomies here presented, important improvements in the state of art are obtained.

It is achieved a device that eases the handling of the patch without the patient has a direct contact with the first surface of the same in which the adhesive is, so it is avoided on one side the damage of such adhesive, because it doesn't come into contact with the corporal greases existing in the fingers and, on the other side it is also avoided the possible removal of parts of the same by being adhered to the fingers of the patient.

Therefore, a correct and perfect adhesion of the patch is guaranteed, increasing in this way the duration of the effectivity of the same.

On the other side, it is eased the fitting process of the patch to the patient, who easily can fit it on a first attempt, avoiding stress situations.

Furthermore, avoiding the contact with the patch, it is obtained a maximum hygiene in the placing process, which is very important due that being about the cares of a stoma, which is an internal part of the body of the patient, communicated to the outer, so it is necessary a maximum cleanliness.

Finally, to the easiness of use, and the efficiency that presents in the placement of these patches, must be added the comfortability of being able to reduce the size of this device, so it doesn't occupy much space and can be considered for example, due also its reduced cost, the sale of the same in the same package that the patches are commercialized.

## Claims

1. Holding device (1) of a patch (2) for ostomies, where such patch (2) is made up by a flat body with a first and second sides (3.1,, 3.2) opposed to each other and a first central hole (4), where the first side (3.1) presents an adhesive and it is apt to fix to the skin of the abdomen of a patient and the second side (3.2) comprise means suitable for holding a picking bag, fixed around the first hole (4) and formed by a circular element (6) with a second central hole (7) concentric with the first hole (4) and a peripheral area (8) separated from the second side (3.2) of the patch (2) along all the outline of the circular element (6), which is defined between an outer diameter (D2) and an intermediate diameter (D1) of such circular element (6), **characterized in that** it comprises
- a holding element (9) longitudinal, that presents a laminar flat shape, with a first and a second ending (10.1, 10.2) opposed;
- two arms (11) longitudinal with flat laminar shape, which present a first ending (12.1) of union of both arms and a second ending (12.2) free, where both arms (11) are arranged symmetrically and are suitable to fit between the second side (3.2) of the patch (2) and the peripheral area (8) of the circular element (6), being the separation space between such endings (12.2) free bigger or equal than the intermediate diameter (D1) of the circular element (6) and smaller that the outer diameter (D2) of the same, and;
- an union means of the first ending (10.1) of the holding device (9) with the first ending (12.1) of union of both arms (11), suitable to permit a first position of use in which the holding element (9) and both arms (11) are contained in a same plane and present a same symmetry axis, being the second ending (10.2) of the holding device (9) opposed to the second ending (12.2) of the arms (11).

2. Holding device (1) of a patch (2) for ostomies, according to claim 1, **characterized in that** the union means are made up by a groove (13) arranged in the first ending (10.1) of the holding element (9), where such groove (13) has a perpendicular straight shape to the symmetry axis and a length equal to the width of the holding element (9) such as the holding device (1) presents a second position in which the holding element (9) is arranged with a turn of 180° regarding the first position and it is arranged on the arms (11) longitudinal, contained in a parallel plain to the same.

3. Holding device (1) of a patch (2) for ostomies, according to claim 1, **characterized in that** the union means are formed by two openings (15) longitudinal arranged in both arms (11) respectively next to the first ending (12.1) of union of both and, two tabs (16) arranged in both laterals o the first ending (10.1) of the holding element (9), suitable to couple in one of the openings (15) of the arms (11) respectively.

4. Holding device (1) of a patch (2) for ostomies, according any of the previous claims, **characterized in that** both arms (11) present a straight section (14) parallel between them, from a section next to the first ending (12.1) until the second ending (12.2) free.

5. Holding device (1) of a patch (2) for ostomies, according to any of the previous claims 1 to 3, **characterized in that** both arms (11) present a shape of circular arch.
